# FASCICULE DE BREVET EUROPEEN

(11) **EP 2 032 629 B1**
(45) Date de publication et mention de la délivrance du brevet: **22.03.2017**
(21) Numéro de dépôt: 07721844.4
(22) Date de dépôt: 15.06.2007
(51) Int. Cl.: C08H 1/06, A61L 27/20, A61L 27/24, A61L 27/26, A61K 8/73, A61Q 19/00, A61K 47/42, C08B 37/00, C08B 37/08, C08L 5/08, C08L 89/00, C08L 89/06

(54) **PRODUIT COLLAGENE POUR APPLICATIONS MEDICALES OU COSMETIQUES, MODE DE FABRICATION ET SES UTILISATIONS**
KOLLAGENPRODUKT FÜR MEDIZINISCHE ODER KOSMETISCHE ANWENDUNGEN, DESSEN HERSTELLUNG UND VERWENDUNGEN
COLLAGEN PRODUCT FOR MEDICAL OR COSMETIC APPLICATIONS, METHOD FOR THE MANUFACTURE THEREOF AND USE THEREOF

(30) Priorité: 22.06.2006 CZ 20060409
(43) Date de publication de la demande: 11.03.2009
(73) Titulaire: Hypro s.r.o, 763 61 (CZ); Biora S.R.O., 69501 Hodonín (CZ)
(72) Inventeur: GALATÍK, Antonín, 76502 Otrokovice (CZ); GALATÍK, Jan, 69601 Rohatec (CZ)
(74) Mandataire: Kreizlova, Dana
(86) Numéro de dépôt international: PCT/CZ2007/000055
(87) Numéro de publication internationale: WO 2007/147369

(56) Documents cités:
- EP-A- 0 296 078
- EP-A- 0 640 647
- CZ-A3- 9 102 025
- FR-A- 2 585 576
- US-A- 5 470 911
- GALATIK A ET AL: "Pharmaceutical preparation based on hyaluronic acid alkali metal salt complexes with multivalent metals" 12 septembre 1989 (1989-09-12), CA , XP002436886 abrégé

## Description

### Domaine technique

L'invention concerne un produit collagène pour applications médicales ou cosmétiques comme les pansements curatifs ou hémostatiques, les membranes de séparation implantables, les feuilles et membranes pour la régénération osseuse et tissulaire guidée, les porteurs solides ou injectables de principes actifs pharmacologiques ou cosmétiques ou les produits cosmétiques et les masques faciaux. En outre,

l'invention concerne le mode de fabrication du produit collagène susmentionné et ses utilisations.

### Etat actuel des connaissances techniques

Pour la préparation de produits cosmétiques, curatifs et hémostatiques, de membranes de séparation et de porteurs de principes actifs pharmacologiques, on utilise de nombreux polymères qui, en contact avec le tissus vivant, ont une compatibilité acceptable et sont biologiquement dégradables après une période acceptable.

Parmi les polymères qui entrent en ligne de compte, le collagène occupe une place privilégiée. Celui-ci, tout d'abord débarrassé des télopeptides - par exemple par le procédé décrit dans le brevet CZ 276891, est très bien toléré dans le tissu vivant et se distingue par une activité hémostatique importante et favorise la guérison. Dans certains cas, la vitesse de résorption des collagènes natifs est trop rapide et afin de parvenir au résultat voulu, il est utile de la ralentir. Dans ce but, il a été déjà auparavant adopté la méthode dite de la réticulation du collagène par des liaisons intramoléculaires transversales covalentes en utilisant les aldéhydes. Plus tard, ces procédés ont été abandonnés car il s'est avéré que les collagènes réticulés par les aldéhydes implantés dans le tissus vivant se calcifient et se transforment peu à peu en formations minérales solides indésirables.

Pour prolonger la période de résorption, des fils chirurgicaux collagènes, par exemple la corde en boyau "cat gut", sont couramment utilisés avec de bons résultats, leur réticulation par des sels chromés. L'utilisation des sels métalliques, les plus souvent chromés, d'aluminium, de zirconium et de titane pour la réticulation du collagène de la peau est une pratique connue et largement utilisée dans l'industrie de la tannerie. Quelquefois, la radiation ultraviolette (UV) est aussi utilisée pour la réticulation du collagène, cependant elle n'augmente que de peu la résistance de la substance collagène irradiée à la dégradation avec des collagénases. L'apparition de radicaux libres pouvant dégrader divers composants actifs est un inconvénient pouvant par exemple dépolymériser les glucosaminoglycannes. En pharmacie, des agents réactifs organiques comme les isocyanates, les composés vinyles ou les carboimides sont utilisées.

Cependant, ce procédé introduit dans le tissage des dérivés étrangers qui ne sont pas propres à l'organisme, leur métabolisme n'est pas tout à fait éclairci et donc une possibilité éventuelle d'effets secondaires indésirables n'est pas exclue.

La toxicité des restes ou des produits non réagis se produisant lors de la libération progressive des agents réticulants organiques utilisés est aussi un inconvénient.

Nombreuses étaient aussi les expériences d'introduire diverses substances actives biologiques dans la matrice collagène, dont parmi elles, les glycosaminoglycannes occupent une place privilégiée, principalement l'hyaluronane qui influence favorablement la guérison des plaies. Par exemple, un complexe de gélatine et de deux glycosaminoglycannes, l'hyaluronane et le sulfate de chondroïtine a été décrit (voir Bychov S.M., Nikolajeva S.S., Charlamova V.N. dans Biull. Eksp. Biol. Med., 1976; 82(10), p. 1211-1213). Principalement l'hyaluronane provoque une augmentation favorable de migration et de prolifération des fibroblastes cutanés. Les produits composés d'hyaluronane et de collagène natif font l'objet de nombreuses demandes de brevets, par exemple la demande de brevet CZ PV 1991-2025.

Les hyaluronanes et même les glycosaminoglycannes peuvent être dimérisés ou réticulés par divers agents avant leur utilisation dans les produits médicaux, cosmétiques et autres. Parmi eux sont connus les dérivés des complexes métalliques. Leur préparation et leur utilisation pharmaceutique sont sujet de nombreux procédés brevetés, par exemple selon les brevets CZ 264 719 ou 267 079. De cette manière, il est possible d'agrandir les macromolécules et ainsi soutenir leur effet physiologique.

L'inconvénient de toutes les solutions à base de mélanges de biopolymères est l' extraction progressive des glucosaminoglycannes de la matrice collagène ou de gélatine si bien que les implants ou les pansements perdent progressivement leur activité respective. Pour améliorer la constance des composites, il a été proposé de lier les deux biopolymères par des liaisons chimiques à l'aide de divers agents synthétiques organiques, par exemple l'hexaméthylènediisocyanate (voir par ex. Bakoš D., Jorge-Herrero E., Koller J. v Polim Med. 2000, 30 (3-4), 57-64), ou 1-ethyl-3-(3-dimethy inaminopropyl), et le carbodiimide (voir Hong S.R., Chong M.S., Lee S.B., Lee Y.M., Song K.W., Park M.H., Hong S.H. dans J. Biomater Sci Polym Ed. 2004; 15(2):201-214).). Il est vrai que de cette manière il est possible de parvenir à des composites constants, mais la question des possibles effets secondaires indésirables des métabolites se produisant des substances réticulantes organiques étrangères utilisées ainsi que du problème de la toxicité chez les dérivés vinyles et même de la cancérigénité des restes non réagis des agents réticulants reste ouverte.

Le produit collagène selon l'invention permet de surmonter les inconvénients susmentionnés dans les applications médicales et cosmétiques. Le principe de l'invention consiste en ce que le produit contient des biopolymères du groupe des glycosaminoglycannes liés à la substance collagène du moins en partie par des liaisons chimiques transmises par les cations des métaux polyvalents.

Les liaisons chimiques citées peuvent être avantageusement transmises par des composés métalliques liés en coordination du groupe des éléments de transition, principalement Cr, Zr, Al, Fe, Mn, Ti et/ou avec les cations métalliques polyvalents des sols alcalins, principalement Ca, Mg, Zn, Ba, Sr.

Les liaisons chimiques des substances collagènes et biopolymères transmises par les cations métalliques polyvalents peuvent être aussi combinées avec des liaisons d'agents réticulants organiques comme les aldéhydes ou les tannins.

L'objet susmentionné est le résultat de nos propres recherches sur le mécanisme de réticulation, de greffe de biopolymères par les liaisons de complexes métalliques. L'importante acquisition constatée concerne la capacité des cations polyfonctionnels des métaux polyvalents de créer des liaison de coordination et des tremplins de complexes métalliques liant le collagène avec les glucosaminoglycannes par une liaison chimique forte. Une telle liaison est indiquée comme une réticulation interpolymère, greffe ou copolymèrisation. Analytiquement, cela prouve une réduction de la teneur en glucosaminoglycannes extractibles des composites collagènes greffés par les complexes métalliques (voir tableau 1 dans la partie des exemples). A partir de ces données, il est évident que le type de cation utilisé influence considérablement la force de la liaison de coordination. Parmi les métaux testés, les cations chromiques produisaient la plus grande stabilité des composites créés, voire à la teneur la plus faible en glucosaminoglycanne extractible.

Parce que les cations des métaux polyvalents représentatifs de ces nouveaux agents greffants inorganiques font souvent même partie des composés constituant les composants cellulaires naturels et les tissus des organismes vivants, leur métabolisme est dans la majorité des cas bien connu et analytiquement facilement suivi. Lors de ces études, il a été aussi constaté que la présence de glucosaminoglycannes dans le collagène provoque dans des cas déterminés une inhibition d'ossification des ligaments de la cuisse des dindons (voir graphe 1 dans la partie concernant les exemples). Le complexe collagène-glucosaminoglycanne résout donc même le problème de calcification non désirée chez les collagènes réticulés par les aldéhydes.

En exploitant ces acquisitions, principalement la faculté des groupes carboxyles des acides aminés "acides" dans le collagène et dans les carboxyles d'acide glucuronique présents dans l'hyaluronane de former des liaisons de coordination fortes avec les atomes des composés hydratés des métaux polyvalents, il est possible de surmonter les deux désavantages principaux des procédés connus jusqu'à présent.

Le nouveau procédé neutralise la tendance de calcification non désirée du collagène réticulé par les aldéhydes dans les implants, mais surtout résout nouvellement la manière de formation des liaisons transversales intramoléculaires et même intermoléculaires physiologiquement plus sûres, ce qui est significatif particulièrement pour les composites pharmaceutiques et cosmétiques. Les réactions réticulantes et greffantes ne sont pas ici transmises par des agents organiques étrangers comme c'est le cas des procédés connus jusqu'à présent, mais par des ions inorganiques qui sont physiologiquement et métaboliquement propres aux organismes vivants. Il est même possible d'opter avantageusement pour des ions métalliques polyvalents indispensables à l'organisme, c.à.d. essenciels et ceux qui font souvent défaut dans le métabolisme.

### Exemple de réalisation de l'invention

Dans l'exemple de réalisation, le produit selon l'invention contient du collagène, dont la structure protéinique est liée, par l'intermédiaire des groupes carboxyles, à un glucosaminoglycanne bioplymère par liaison de coordination avec les ions métalliques. Sous l'appellation de glucosaminoglycanne, nous entendons le groupe de biopolymères contenant répétitivement de la glucosamine liée par liaison glycosidique avec n'importe quel glyco-amino acide simple, et, dans le cas de l'hyaluronane avec de l'acide glucuronique. Le produit peut même contenir des substances auxiliaires, comme les produits de régulation de la viscosité, les produits hydratants, les agglomérats, les adoucisseurs, les accélérateurs de pénétration, les agents de conservation et de désinfection, les régulateurs de pH, les antioxydants, les stabilisateurs de principes actifs, les huiles, les graisses, les cires, les émulsifiants, les substances aromatiques, les colorants et/ou les masses de remplissage inertes.

Dans l'exemple de réalisation, le collagène du produit est de type I, tout au moins partiellement débarrassé des télopeptides, sa structure n'est pas réticulée de manière significative. Il contient un glycosaminoglycanne lié -l'hyaluronane de sodium- dont la liaison de coordination est transmise par les sels de magnésium.

Le produit collagène peut être sous forme de poudre, de microparticules, de fibres, de flocons, de mousse, de matière feutre, d'éponge, d'aiguilles, de bâtonnets, de tablettes, de gel, de solution visqueuse, de crème, de feuille ou de stratifié.

Le procédé de fabrication du produit collagène dans l'exemple de réalisation comprend le séchage dans un séchoir à pulvérisation, le séchage par congélation, le revêtement ou le coulage suivi de séchage, les processus de séparation et la précipitation des phases, le remplissage dans les récipients, les récipients sous pression y compris pour la formation de mousses et/ou d'aérosols.

Les produits collagènes contenant des glycosaminoglycannes liés sont, selon l'état de la technique, fabriqués en règle générale par réticulation et par liaison covalente de composés biopolymères par des agents réactifs organiques contenant des groupes isocyanates, vinyles ou carboimides, ou encore par radiation UV. A l'inverse, pour la liaison et/ou la réticulation des biopolymères d'après le produit selon l'invention, sont utilisés des réactions de complexes métalliques dans lesquelles les groupes carboxyles des biopolymères se posent en ligand donneur et les cations des métaux polyvalents hydratés, par avantage hydroxylés, sont receveurs de la paire électronique du carboxyle lors de la formation très stable de la dite liaison de coordination. Parce que les protéines contiennent beaucoup de groupes carboxyles libres appartenant aux chaînes secondaires des restes aminoacides d'acide asparagique et glutamique, la réaction est très rapide et il est utile de la retarder par diminution du pH, lequel réprime la dissociation et la réactivité des groupes carboxyles. De cette manière se sont formées des liaisons intermoléculaires transversales tout comme un "greffement" extramoléculaire, c.à.d. une liaison réciproque des chaînes polymères des biopolymères individuels. Il est possible de réguler très simplement le degré de réticulation transversale du collagène. Lors d'une réaction du pH sous une valeur aux alentours de 2,0, il ne se produit principalement qu'une liaison des ions métalliques en un point et une réticulation quasi nulle du collagène, ce qui est visible au fait qu'il ne se produise pas d'augmentation de sa température dénaturante. A des fins pharmaceutiques et cosmétiques, une réticulation basse est appropriée, élevant la température dénaturante de la transformation du collagène en gélatine d'environ 1 à 5°C.

La démarche essentielle pour la formation d'un glucosaminoglycanne lié en coordination est la liaison du cation receveur polyfonctionnel du métal polyvalent par l'intermédiaire des groupes carboxyles des éléments sacharides acides et des acides aminés, l'acide glutamique et asparagique dans le collagène. La solution de sels métalliques utilisée devrait avoir un taux de pureté élevé et une teneur faible en sels neutres. La réaction se passe ordinairement dans un milieu faiblement acide et est suivie d'une augmentation de la viscosité et dans le cas d'un degré supérieur de réticulation par l'apparition d'un précipité fibreux. La démarche importante suivante est la réaction du collagène contenant les glucosaminoglycannes liés avec un surplus supplémentaire de collagène. Elle s'effectue avantageusement par ajout lent d'une suspension du complexe déjà créé à une solution colloïdale de collagène natif tout en mélangeant. Tout d'abord, se produit une précipitation du mélange réactionnel, mais d'autres additifs supplémentaires mènent à sa dispersion progressive et à la formation d'une solution visqueuse colloïdale.

Les différences dans le degré de réticulation du collagène, dans la teneur en hyaluronane lié et dans la teneur éventuelle en collagène libre ont une influence considérable sur les propriétés du produit collagène, par ex. elles influencent la stabilité du gel et de la mousse et de la vitesse de résorption de l'implant. Par le choix du type d'ions des complexes métalliques, de la teneur en hyaluronane et du degré de réticulation, il est possible en larges termes de réguler sans à-coups les propriétés du produit selon les exigences requises à son utilisation.

Le produit collagène dans l'exemple de réalisation selon l'invention trouve son application prioritairement comme hémostatique résorbant avec une durée de résorption prolongée, comme membrane implantable résorbante pour la régénération osseuse et tissulaire guidée, pour la séparation des tissus et la constitution de barrières d'agglutination lors des opérations chirurgicales, comme pansement curatif pour le soin des lésions cutanées et comme porteur de principes actifs pharmacologiquement permettant une conduite progressive et ciblée de la cinétique de leur libération.

Comme principes actifs fournis par le produit collagène selon l'invention font partie par ex. les antibiotiques, les antiseptiques, les anesthésiques, les analgésiques, les cytostatiques, les hormones, les stéroïdes, les cytokines, les stimulants et les facteurs pour la libération et l'inhibition de la libération des hormones, des prostaglandines, des enzymes, et des facteurs de croissance et ostéoinductifs.

### Domaines d'utilisation de l'invention

Le produit collagène selon l'invention est applicable sur la peau sous forme de produits cosmétiques, de masques faciaux mousseux ou de feuilles pour le soin par exemple de peaux vieilles, ridées ou sales, pour le soin et la protection de l'épiderme exposé en milieu ou à des radiations défavorables.

Le produit collagène peut aussi contenir d'autres substances auxiliaires:
- hydratantes, comme les glycérines, le sorbitol, le polyéthylène glycol, le polypropylène glycol ou les glycosaminoglycannes libres;
- adoucissantes, comme par ex. les esters d'acide citrique, vinique ou glycérique;
- de conservation, comme les dérivés du crésol, l'alcool phénéthylique, le chlorbutanol, le méthyl- et éthyl- ou propyl-ester d'acide hydroxybenzoïque, le chlorure de benzalconium, le chlorure de cétylpyridinium, le diacétate et digluconate de chlorhexidine, l'éthanol, l'isopropanol, le propylène glycol.
- Les produits désinfectants, comme l'iode, le brome, l'iode polyvidone, les composés halogènes comme le chlorure de sodium, l'hypochlorite de sodium, les produits oxydants comme l'eau oxygénée (peroxide d'hydrogène), le permanganate de potassium, les composés aryl et alkyl du mercure, les composés organostanniques comme le tri-n-butylétain benzoate, les composés d'argent comme l'acétyltannate d'argent protéine, les alcools, les phénols ou les composés organiques d'azote comme 8-hydroxyquinoline, le chloroquinaldol, le clioquinol, l'ethacridine, l'hexetidine, la chlorexidine, l'ambazone;
- Les régulateurs de pH comme les tampons de citrate-borate-phosphate et leurs composés
- Les antioxydants comme l'acide ascorbique, le palmitate d'ascorbyle, l'acétate de tocophérol, le propyl galatte, le butylhydroxyanisole ou le butylhydroxytoluène
- Les stabilisateurs de substances actives comme le mannitol, le glucose, le lactose, le fructose et le saccharose
- Les substances auxiliaires émulsifiables comme les huiles, les graisses et les cires
- Les substances odorantes, les colorants, les agents purifiants, les produits pour le soin de la peau
- Les stabilisants d'émulsion comme les émulgateurs non-ionogéniques, amphotériques, cationiques et les tensides anioniques
- Les masses de remplissage comme la cellulose microcristalline, l'oxyde d'aluminium, l'oxyde de zinc, l'oxyde de titane, le talc, l'oxyde de silicium, silicate de magnésium, magnésium aluminosilicate, le kaolin, les dérivés d'amidon, stéarate de zinc ou de calcium et le phosphate de calcium.

Il est possible d'introduire les substances actives et auxiliaires dans le produit selon l'invention de manière que la substance se dissolve ou se disperse dans la dispersion du collagène terminée avec un hyaluronane lié encore avant le processus de formation. S'il est utilisé plusieurs substances, il est possible d'introduire certaines de celles-ci après le processus de formation par enrobage, vaporisation, imprégnation, immersion ou par d'autres processus d'absorption.

Lors de l'utilisation du produit collagène selon l'invention pour application dermale, intradermale et transdermale des substances pharmaceutiquement ou cosmétiquement actives, il est donné la priorité aux formes plates de réalisation, comme les feuilles, les membranes, les mousses ou les éponges. Ces formes plates peuvent avoir une structure stratifiée formée par des couches de séparation sans substances actives, des couches de séparation perméables, des membranes de régulation et une couche de colle. Ces produits collagènes uni- ou multi-couches sont revêtus prioritairement d'une couche-arrière et, du côté opposé, d'une couche de protection détachable, lesquelles sont composées d'un matériau courant pour un spécialiste comme ceux qui s'utilisent pour la fabrication par ex. de pansements ou de rubans adhésifs.

Dans la réalisation du produit collagène selon l'invention destiné aux plaies superficielles et même intracorporelles, la forme du produit est poreuse, par ex. mousseuse, en feutre fibreux ou spongieuse. La grandeur et la structure du produit sont choisies de manière à permettre la pénétration des cellules, par ex. des fibroblastes ou des ostéoblastes dans la structure du produit et leur orientation d'une manière semblable comme pour les tissus ligamenteux collagènes.

**Tableau 1: contenance d'hyaluronane extractible par l'eau dans 500 mg de composite collagène. (documente la réduction de la contenance de glucosaminoglycannes extractibles des composites de complexes métalliques collagènes greffés).**

| | Quantité d'hyaluronane (mg) | | | |
|---|---|---|---|---|
| Contenance d'hyaluronane rajouté dans du gel avec 500 mg de collagène | 0 | 25 | 50 | 100 |
| Rendement d'extraction d'un échantillon non-réticulé | 0 | 22 | 47 | 93 |
| Rendement d'extraction d'un échantillon réticulé de Mg²⁺ (10 mg MgO) | 0 | 0 | 8 | 14 |
| Rendement d'extraction d'un échantillon réticulé de Cr³⁺ (10 mg Cr₂O₃) | 0 | 0 | 0 | 7 |

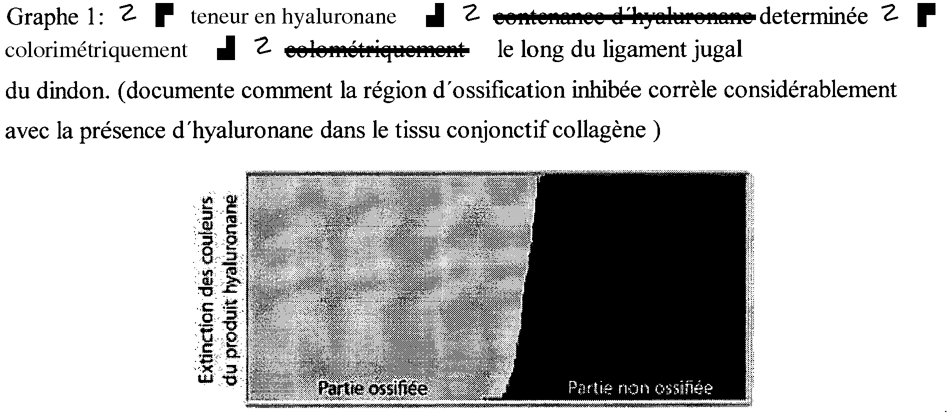

## Revendications

1. Produit collagène pour application médicale ou cosmétique, se distinguant par la présence de biopolymères du groupe de glycosaminoglycannes, liée à la substance collagène du moins en partie par des liaisons chimiques transmises par les cations des métaux polyvalents.

2. Le produit collagène selon la revendication 1, se distinguant par le fait que sa substance collagène et/ou glycosaminoglycanne est chimiquement réticulée.

3. Le produit collagène selon les revendications 1 et 2, se distinguant par le fait que la structure moléculaire de sa substance collagène est débarrassée des télopeptides.

4. Le produit collagène selon les revendications 1 à 3, se distinguant par le fait que la structure moléculaire spatiale de sa substance collagène est du moins en partie transformée en structure désordonnée, dite conformation gélatineuse.

5. Le produit collagène selon la revendication 1, se distinguant par le fait que le glycosaminoglycanne biopolymère est un sel sodique d'acide hyaluronique et/ou ses dérivés.

6. Le produit collagène selon la revendication 1, se distinguant par le fait que les liaisons chimiques sont transmises par des composés liés en coordination de métaux du groupe des éléments de transition et/ou avec les cations polyvalents des métaux de sol alcalin.

7. Le produit collagène selon la revendication 1, se distinguant par le fait que les liaisons chimiques de la substance collagène et biopolymère transmises par les cations de métaux polyvalents sont combinées avec des liaisons d'agents réticulants organiques

8. Le produit collagène selon la revendication 1, se distinguant par le fait qu'il contient des substances auxiliaires comme les produits de conservation, les désinfectants et les produits bactériostatiques, les antibiotiques, les régulateurs de pH, les antioxydants, les substances actives pharmacologiquement et/ou cosmétiquement, les huiles, les graisses, les cires, les stabilisants d'émulsion, les substances odorantes, les colorants ou les masses de remplissage.

9. Le produit collagène selon les revendications 1 à 8, se distinguant par le fait qu'il est sous la forme/consistance de poudre, de fibres, de flocons, de mousses, de tissus, de tricots, de feutres non fibré, d'éponges, de bâtonnets, de tablettes, de gels, de crèmes, d'objets formés spatialement, de feuilles monocouches ou de stratifiés et /ou est porté ou imprégné dans des matériaux textiles tissés, tricotés ou non tissés sur base de fibres naturelles et/ou synthétiques.

10. Le mode de fabrication du produit collagène selon la revendication 1, se distinguant par le fait que la matière première collagène est tout d'abord débarrassée des protéines non collagènes par extraction progressive dans des solutions acides et alcalines, puis, par lavage, les composés solubles et les sels sont retirés, ensuite, par désintégration mécanique, la matière première est morcelée et dissoute dans un milieu acide en gel colloïdal, après quoi, une solution de sulfate de magnésium et d'hyaluronane est incorporée dans la partie séparée du gel et en mélangeant doucement se laisse réagir jusqu'à l'apparition d'une suspension fibreuse fine qui se sépare avantageusement par centrifugation et, par rinçage répété, est débarrassée des composés de magnésium non réagis et des sels de sulfate, puis est incorporée dans la solution restante de collagène, si bien qu'elle forme un gel visqueux dans lequel, et dans un milieu de pH contrôlé, se déroule lentement la récupération et une réaction de coordination suivante entre les groupes carboxyles de l'hyaluronane et les complexes métalliques liés au collagène, juste après quoi, après achèvement de ces processus, le produit préparé se sèche par congélation ou par atomisation, s'applique ou se moule avec séchage s'ensuivi, se sépare ou se rempli dans des récipients pour la formation de poudres, de flocons, de fibres, de mousses, d'éponges, de bâtonnets, de tablettes, de gels, de crèmes, de feuilles unicouches, ou de stratifiés.

11. Le mode selon la revendication 10, se distinguant par le fait que s'ajoute dans la dispersion collagène des substances auxiliaires, comme les adouccisseurs, les produits de conservation, les produits de désinfection et bactériostatiques, les régulateurs de pH, les antioxydants, les substances actives pharmaceutiquement, les stabilisants de substances actives, les émulsifiants, les substances odorantes, les colorants, ou les masses de remplissage.

12. L'utilisation du produit collagène selon l'une des revendications 1 à 8, se distinguant par le fait qu'il consiste à la préparation de bandages destinés à la guérison des lésions cutanées, des ulcères variqueux et comme pansement provisoire après prélèvement de greffe de peau et/ou à des fins hémostatiques.

13. L'utilisation du produit collagène selon l'une des revendications 1 à 8, se distinguant par le fait qu'il consiste à la préparation de barrières unicouches ou stratifiées pour la régénération tissulaire et osseuse guidée.

14. L'utilisation du produit collagène selon l'une des revendications 1 à 8, se distinguant par le fait qu'il consiste à la préparation de produits pharmaceutiques destinés à l'implantation ou à l'injection dans l'organisme vivant.

15. L'utilisation du produit collagène selon l'une des revendications 1 à 8, se distinguant par le fait qu'il consiste à la préparation de produits pharmaceutiques pour une libération guidée de substances actives.

16. L'utilisation du produit collagène selon l'une des revendications 1 à 8, se distinguant par le fait qu'il consiste à la préparation de produits cosmétiques pour une libération guidée de substances actives.

## Patentansprüche

1. Kollagenprodukt zur medizinischen oder kosmetischen Anwendung, **dadurch gekennzeichnet, dass** den Zusatz von Biopolymeren aus der Gruppe der Glykosaminoglykane, die zumindest teilweise über von Kationen mehrwertiger Metalle übertragene chemische Verbindungen mit der Kollagensubstanz verbunden sind.

2. Kollagenprodukt laut Anspruch 1, **dadurch gekennzeichnet, dass** die Kollagen- und/oder Glykosaminoglykansubstanz chemisch vernetzt wurde.

3. Kollagenprodukt laut Anspruch 1 und 2, **dadurch gekennzeichnet, dass** die Molekularstruktur der Kollagensubstanz von Telopeptiden befreit wurde.

4. Kollagenprodukt laut Anspruch 1 bis 3, **dadurch gekennzeichnet, dass** die spatiale Molekularstruktur der Kollagensubstanz zumindest teilweise in eine ungeordnete Struktur (gallertartige Masse) umgewandelt wurde.

5. Kollagenprodukt laut Anspruch 1, **dadurch gekennzeichnet, dass** das Glykosaminoglykan-Biopolymer ein Natriumsalz der Hyaluronsäure und/oder ein Derivat davon ist.

6. Kollagenprodukt laut Anspruch 1, **dadurch gekennzeichnet, dass** die chemischen Verbindungen durch verwandte Verbindungen in Koordination mit Metallen aus der Gruppe der Übergangselemente und/oder mit den mehrwertigen Kationen der Natriumsalzmetalle übertragen werden.

7. Kollagenprodukt laut Anspruch 1, **dadurch gekennzeichnet, dass** die von den Kationen mehrwertiger Metalle übertragenen chemischen Verbindungen der Kollagen- und Biopolymersubstanz mit Verbindungen von organischen Vernetzungsmitteln kombiniert werden.

8. Kollagenprodukt laut Anspruch 1, **dadurch gekennzeichnet, dass** es Hilfssubstanzen wie Konservierungsmittel, Desinfektionsmittel und bakteriostatische Substanzen, Antibiotika, pH-Regulatoren, Antioxidantien, pharmakologische und/oder kosmetische Wirkstoffe, Öle, Fette, Wachse, Emulsionsstabilisatoren, Duftstoffe, Farbstoffe oder Füllstoffe enthält.

9. Kollagenprodukt laut Anspruch 1 bis 8, **dadurch gekennzeichnet, dass** es in Form/Konsistenz von Puder, Fasern, Flocken, Schaum, Gewebe, Gewirk, faserfreiem Filz, Schwämmen, Stäbchen, Tabletten, Gel, Creme, spatial geformten Objekten, einschichtigen Folien oder Hartgewebe vorliegt und/oder in gewebte, gewirkte oder nicht gewebte Materialien auf Basis von Naturfasern und/oder synthetischen Fasern eingebracht oder imprägniert wird.

10. Herstellungsmethode des Kollagenproduktes laut Anspruch 1, **dadurch gekennzeichnet, dass** der Kollagenrohstoff zunächst durch progressive Extraktion in Säuren und Laugen von nicht kollagenen Proteinen und danach durch Waschen von löslichen Verbindungen und Salzen befreit wird; danach wird der Rohstoff durch mechanische Zerstörung zerkleinert und in Säure zu einem kolloidalen Gel aufgelöst, woraufhin eine Magnesiumsufat- und Hyaluronanlösung in den separierten Teil des Gels eingebracht und durch sanftes Mischen eine Reaktion abgewartet wird, bis eine feinfaserige Suspension entsteht, die sich durch Zentrifugieren abtrennt und durch mehrmaliges Spülen von verbliebenen Magnesiumverbindungen und Natriumsalzen befreit wird; danach wird diese in die verbliebene Kollagenlösung eingebracht, sodass sich ein viskoses Gel bildet, in dem bei einem kontrollierten pH-Wert langsam eine Rückgewinnung und eine darauf folgende Koordinationsreaktion zwischen den Carboxylgruppen des Hyaluronans und den an das Kollagen gebundenen Metallkomplexen abläuft; hierauf trocknet das vorbereitete Produkt durch Tiefkühlung oder Atomisierung, wird bei anschließender Trocknung aufgetragen oder geformt, getrennt oder in Behälter gefüllt, um Puder, Flocken, Fasern, Schaum, Schwämme, Stäbchen, Tabletten, Gels, Cremes, einschichtige Folien oder Hartgewebe zu bilden.

11. Herstellungsmethode laut Anspruch 10, **dadurch gekennzeichnet, dass** bei der Kollagendispersion Hilfssubstanzen wie Weichmacher, Konservierungsmittel, Desinfektionsmittel und bakteriostatische Substanzen, pH-Regulatoren, Antioxidantien, pharmakologische Wirkstoffe, Emulsionsstabilisatoren, Emulgatoren, Duftstoffe, Farbstoffe oder Füllstoffe hinzugefügt werden.

12. Verwendung des Kollagenproduktes laut einem der Ansprüche 1 bis 8, **dadurch gekennzeichnet, dass** es zur Herstellung von Bandagen für die Heilung von Hautwunden, varikösen Geschwüren und als provisorischer Verband nach der Entnahme von Hauttransplantaten und/oder zur Blutstillung dient.

13. Verwendung des Kollagenproduktes laut einem der Ansprüche 1 bis 8, **dadurch gekennzeichnet, dass** es zur Herstellung von einlagigen Auflagen oder Hartgeweben für die gesteuerte Gewebe- und Knochenregeneration dient.

14. Verwendung des Kollagenproduktes laut einem der Ansprüche 1 bis 8, **dadurch gekennzeichnet, dass** es zur Herstellung von pharmazeutischen Produkten für die Implantation oder Injektion in den lebenden Organismus dient.

15. Verwendung des Kollagenproduktes laut einem der Ansprüche 1 bis 8, **dadurch gekennzeichnet, dass** es zur Herstellung von pharmazeutischen Produkten für eine gesteuerte Abgabe von Wirkstoffen dient.

16. Verwendung des Kollagenproduktes laut einem der Ansprüche 1 bis 8, **dadurch gekennzeichnet, dass** es zur Herstellung von kosmetischen Produkten für eine gesteuerte Abgabe von Wirkstoffen dient.

## Claims

1. Collagen product for medical or cosmetic application, **characterised by** the presence of biopolymers from the glycosaminoglycan group, bonded to the collagen material, at least in part, by chemical bonds transferred by cations of polyvalent metals.

2. The collagen product of claim 1, **characterised by** that its collagen material and/or glycosaminoglycan is chemically reticulated.

3. The collagen product of claims 1 and 2, **characterised by** that the molecular structure of its collagen substance is telopeptide-free.

4. The collagen product of claims 1 to 3, **characterised by** that the spatial molecular structure of its collagen substance is, at least in part, converted to a disordered structure known as gelatine conformation.

5. The collagen product of claim 1, **characterised by** that the glycosaminoglycan biopolymer is a sodium salt of hyaluronic acid and/or its derivatives.

6. The collagen product of claim 1, **characterised by** that chemical bonds are transferred by coordinated compounds of the metals of the transition element group and/or with polyvalent cations of alkaline-soil metals.

7. The collagen product of claim 1, **characterised by** that the chemical bonds of the collagen material and biopolymer transferred by cations of polyvalent metals are combined with the bonds of organic reticulating agents.

8. The collagen product of claim 1, **characterised by** that it contains auxiliary materials such as preservative agents, disinfectants and bacteriostats, antibiotics, pH regulators, antioxidants, pharmacologically and/or cosmetically active substances, oils, fats, waxes, emulsion stabilisers, odorous materials, dyes or filling compounds.

9. The collagen product of claims 1 to 8, **characterised by** that it is in the form/consistency of powder, fibres, vials, mousses, fabrics, knitted fabrics, non-woven felts, sponges, strips, tablets, gels, creams, spatially formed objects, monofilament or layered sheets and/or is worn or impregnated into the woven, knitted or non-woven textile materials made of natural and/or synthetic fibres.

10. The manufacturing method for the collagen product of claim 1, **characterised by** that non-collagenous proteins are first and foremost removed from the collagen raw material by gradual extraction in acidic and alkaline solutions. Soluble compounds and salts are then removed by washing. The raw material is fragmented and dissolved in an acidic medium into a colloidal gel by mechanical disintegration. After this, a solution of magnesium sulphate and hyaluronan is added to the separated part of the gel which, with gentle stirring, reacts to form a fine, fibrous suspension which is separated by centrifugation. Unreacted magnesium compounds and sulphate salts are removed by repeated washing. This is then added to the remaining solution of collagen such that is forms a viscous gel in which, along with a controlled pH medium, the recovery and the following coordination reaction between the carboxyl groups of hyaluronan and the complex metals bonded with the collagen takes places gradually. Immediately after the completion of these processes, the prepared product is dried by freezing or atomisation. It is applied or moulded with drying or is either separated or filled in containers for making powders, vials, fibres, mousses, sponges, strips, tablets, gels, creams, monofilament sheets or layers.

11. The method mentioned in claim 10, **characterised by** that auxiliary materials such as softeners, preservative agents, disinfectants and bacteriostats, antibiotics, pH regulators, antioxidants, pharmacologically active substances, stabilisers of active substances, emulsifiers, odorous materials, dyes or filling compounds are added to the collagen dispersion.

12. The use of the collagen product mentioned in one of claims 1 to 8, **characterised by** that it is used for preparing bandages designed to heal skin lesions, varicose ulcers and as temporary dressing after removing the skin graft and/or for haemostatic purposes.

13. The use of the collagen product mentioned in one of claims 1 to 8, **characterised by** that it is used for preparing monofilament or layered barriers for guided tissue and bone regeneration.

14. The use of the collagen product mentioned in one of claims 1 to 8, **characterised by** that it is used for preparing pharmaceutical products for implantation or injection into a living organism.

15. The use of the collagen product mentioned in one of claims 1 to 8, **characterised by** that it is used for preparing pharmaceutical products for guided liberation of active substances.

16. The use of the collagen product mentioned in one of claims 1 to 8, **characterised by** that it is used for preparing cosmetic products for guided liberation of active substances.
